# EUROPÄISCHE PATENTSCHRIFT

(11) **EP 0 755 677 B1**
(45) Veröffentlichungstag und Bekanntmachung des Hinweises auf die Patenterteilung: **29.12.1999**
(21) Anmeldenummer: 96111829.6
(22) Anmeldetag: 23.07.1996
(51) Int. Cl.: A61K 9/22, A61L 27/00

(54) **Pharmafreisetzende Körperchen**
Drug delivering corpuscles
Corpuscules pour délivrer un médicament

(30) Priorität: 26.07.1995 DE 19527306
(43) Veröffentlichungstag der Anmeldung: 29.01.1997
(73) Patentinhaber: Härle, Anton, Prof. Dr., D-48161 Münster (DE)
(72) Erfinder: Härle, Anton, Prof. Dr., D-48161 Münster (DE)

(56) Entgegenhaltungen:
- WO-A-82/03174
- DE-A- 3 834 944
- GB-A- 1 316 998

## Beschreibung

Die Erfindung betrifft pharmakafreisetzende Körperchen zur lokalen Behandlung und Verwendung im oder auf dem Körpergewebe, bestehend aus einem biologisch inerten Tragermaterial wie z.B. Polymethylmetacrylat, wobei ein oder verschiedene Pharmaka in gleichförmiger Verteilung freisetzbar in dem Trägermaterial vorliegen und die Körperchen miteinander verbunden sind.

Aus der DE-OS 23 20 373 ist ein antibiotikahaltiges Mittel in Kugelform bekannt, bei dem die Kugeln mit Hilfe von Fäden oder Drähten miteinander verbunden sind. Derartige antibiotikahaltige Hilfsmittel werden in weitem Umfang in der Behandlung von Knochen- und Weichteilinfektionen eingesetzt. Der besondere Vorteil liegt darin, daß die eingesetzten Antibiotika direkt in den Infektionsherd eingebracht werden und zum Einsatz kommen; nachteilige Nebenwirkungen auf von der Infektion nicht betroffene Körperabschnitte oder Organe können damit fast vollständig vermieden werden.

In der EP 0 157 909 wurden Körperchen in Form eines Rotationsellipsoids vorgeschlagen. Hiermit war zwar theoretisch eine günstigere Oberflächen/Volumen-Relation zu erreichen, doch stellte sich die Herstellung dieser Form als sehr schwierig heraus. Auch in der klinischen Anwendung konnte mit dieser Ausgestaltung nicht immer die erforderliche Wirkstoffkonzentration im erkrankten Gewebe erreicht, die für den therapeutischen Erfolg erforderlich ist. Eine Steigerung der kontrollierten Pharamakfreisetzung insbesondere hinsichtlich der lokalen Gegebenheiten im Operationsgebiet war daher wünschenswert.

Außerdem ist die Herstellung eines relativ spitz zulaufenden Partikel aus produktionstechnischen Gründen zur Zeit noch mit vielen Schwierigkeiten verbunden.

Fin weiterer Nachteil der bisher bekannten Ausgestaltungen pharmakafreisetzender Körper mit kugelrunder bzw. rotationsellipsoider Ausgestaltung war, daß sie im Gewebe stark auftragen und deshalb z.B. kaum im Bereich direkt unter der Haut liegender Knochenabschnitte eingelegt werden konnten. Auch die Anlagerung der pharmakafreisetzenden Partikel an oder unter Osteosynthesehilfsmittel war wegen ihres kreisrunden Querschnitts erschwert oder behindert.
Aber auch bei der Auffüllung von Knochenhöhlen ließen sich die pharmakafreisetzenden Körper mit kugelrunder bzw. rotationsellipsoider Ausgestaltung nicht dicht packen, sodaß zwischen ihnen große Freiräume verblieben, die nicht zur Antibiotikafreisetzung genutzt werden konnten.

Da die Pharmakafreisetzung ein oberflächenabhängiger Prozeß ist, d.h. daß zunächst nur die in den oberflächlichen Schichten liegenden Pharmaka freigesetzt werden, wird die klinisch relevante Freisetzung in den ersten Anwendungstagen nicht vom im Kern liegenden Pharmakadepot beeinflußt. Kugelige oder in mehreren rechtwinkligen Schnittebenen kreisrunde Körperchen stellen daher immer nur einen Teil der inkorporierten Pharmaka der klinischen Nutzung zur Verfügung.

Der kreisrunde Querschnitt der miteinander zu Ketten verbundenen pharmakafreisetzenden Körperchen gemäß dem Stand der Technik behinderte auch ihre Wiederentfernung, wenn diese Ketten unter Zugwirkung aus dem Körpergewebe entfernt werden mußten, da sich bei der Heilung um die Körperchen Granulationsgewebe gebildet hatte und dann beim Herausziehen der Ketten das Durchrutschen der einen relativ großen Querschnitt aufweisenden Partikel erschwert ist.

Diese der Erfindung zugrundeliegende Aufgabe der Verbesserung der Pharmakafreisetzung, der Erhöhung der lokalen Pharmakonzentration und der leichteren Handhabung wird durch die in den Ansprüchen genannten Merkmale gelöst.

Durch die Ausgestaltung der pharmakafreisetzenden Körperchen mit einem abgeplatteten Querschnitt z.B. im Sinne eines Ellipsoids kann die Pharmakfreisetzung durch Vergrößerung der Oberfläche je Volumeneinheit erheblich erhöht werden. Auf das klinisch sich kaum auswirkende zentrale Pharmakadepot im Quer- oder Längsschnitt kreisrunder Partikel wird bei den vorgeschlagenen Formgestaltungen größtenteils verzichtet und so eine Ökonomisierung hinsichtlich der eingesetzten Pharmakamenge erzielt. Besonders bedeutsam ist dabei aber, daß durch dichtere Packung und günstigere Oberflächen/Volumen-Relation die lokale Pharmakakonzentration gesteigert werden kann, was insbesondere bei der Behandlung von Gewebsinfektionen eine große Bedeutung hat. Die in den letzten Jahren zu beobachtende Zunahme resistenter Infektionserreger erfordert eine möglichst hohe lokale Antibiotikakonzentration, um einen Heilungserfolg zu bewirken.

Die Entwicklung neuer Antibiotika ist in den letzten Jahren mehr oder weniger zum Stillstand gekommen und so kann dem drohenden Überhandnehmen der Antibiotikaresistenz am besten dadurch begegnet werden, daß der Entwicklung resistenter Bakterienstämme dadurch vorgebeugt wird, daß durch initial eine ernorm hohe Wirkstoffkonzentration dieser Prozeß gleich unterbunden wird.

Der Aufgabe liegt daher die Aufgabe zugrunde, pharmakafreisetzende Körperchen zu schaffen, die eine derartige Formgestalt aufweisen, daß dadurch eine maximale Wirkstoffkonzentration im erkrankten Gewebe mittels ihrer operativen Einlage erreicht werden kann, auch in Gewebsarealen , die bisher einer derartigen Therapie kaum zugänglich waren.

Diese der Erfindung zugrundeliegende Aufgabe wird durch die Lehre des Hauptanspruchs gelöst.

Vorteilhafte Ausgestaltungen sind in den Unteransprüchen erläutert.

Mit anderen Worten ausgedrückt, wird vorgeschlagen, pharmakafreisetzende Körperchen mit einem möglichst günstigen Oberfläche/Volumenverhältnis bzw. mit flächiger Formgestalt zu schaffen, die wenig auftragen.

Da die Körperchen nun mehr flächig ausgestaltet sind, tragen sie nicht mehr so auf und können auch an unmittelbar unter der Haut gelegene Knochenabschnitte angelegt werden, ohne daß innere Druckgeschwüre zu befürchten sind.

Auch die Anlagerung an oder unter Osteosynthesehilfsmittel wie Platten, Stäbe und Endoprothesen ist damit erheblich verbessert, da keine wesentliche Schwächung der vorgegebenen Implantate erforderlich ist, um Freiräume für die anzulagernden pharmahaltigen Körperchen zu schaffen. Auch an nahe der Haut liegende Implantate können nun derartige erfindungsgemäße Partikel angelagert werden, ohne daß räumliche Probleme auftreten würden.

Schließlich wird auch die Wiederentfernung aus Weichteil- und Knochengewebe erleichtert, da durch die größere Packungsdichte nun das zwischen den Partikeln liegende Granulationsgewebe weniger stark ausgeprägt ist, das dem Durchrutschen der Partikel einen von der Gewebsdicke abhängigen Widerstand entgegensetzt.

Diese Ausführungsbeispiele der erfindungsgemäßen Formausgestaltung der pharmakafreisetzenden Körperchen werden nachfolgend anhand der Zeichnungen erläutert. Die Zeichnungen zeigen dabei in
Fig. 1 eine Ansicht miteinander als Kette verbundener Partikel,
Fig. 2 bis 5 Längsschnittbilder verschiedener Formgestal tungen,
Fig. 6 bis 9 Querschnittbilder verschiedener Formgestal tungen.

In der Beschreibung und in den Ansprüchen werden folgende Definitionen gebraucht:
**Ketten** bezeichnen durch Fäden/Drähte miteinander verbundene pharmakafreisetzende Körperchen,
**Partikel und Körperchen** bezeichnen die erfindungsgemäßen pharmakafreisetzenden Körperchen als Ganzes,
**Abschnitte** bezeichnen Teile der Körperchen im Längsschnittbild,
**Segmente** bezeichnen die ober- oder unterhalb der Symmetrieachse (Draht/Faden) liegenden Teile der Segmente im Längsschnittbild.

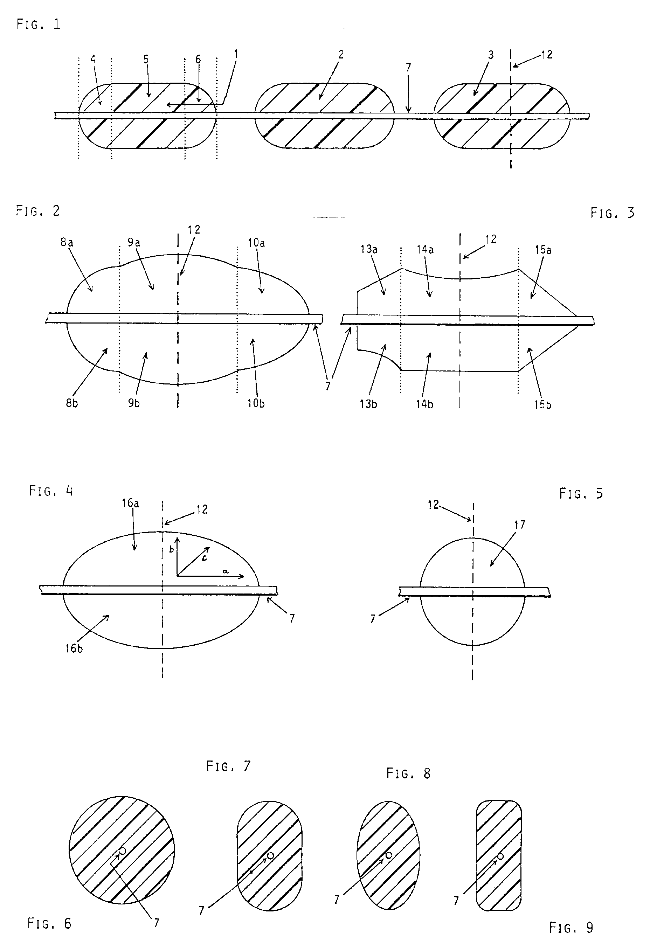

Bei dem in Fig. 1 dargestellten Kettenabschnitt bezeichnen 1, 2 und 3 gleichartige pharmakafreisetzende Körperchen als Kettenglieder, die mittels eines Fadens oder Drahtes (7) miteinander verbunden sind. Die pharmakafreisetzenden Körperchen sind in einem axialen Längsschnittbild dargestellt und weisen an beiden Drahtaustrittsenden eine sich verjüngende Formgestalt auf. Die Körperchen lassen sich in dieser Projektion entsprechend der punktierten Linien in drei Abschnitte unterteilen, wobei 4 und 6 die endständigen Verjüngungsabschnitte bezeichnen, und mit 5 der Mittelabschnitt definiert ist.
Die in Fig. 1 dargestellten Körperchen haben demnach im axialen Längsschnittbild zwei kreisrund ausgestaltete endständige Verjüngungsabschnitte, zwischen die ein rechteckiges Mittelstück geschaltet ist. Wenn die Partikel als Drehkörper um die axiale Längsachse, d.h. um den Draht/Faden 7 geformt werden, besitzen die Verjüngungsabschnitte eine sphärische und der Mittelabschnitt eine zylindrische Form. Ganz andere Formen der erfindungsgemäßen Körperchen entstehen dann, wenn die Rotationsachse nicht zentral sondern exzentrisch verläuft.

Bei besonders vorteilhaften Ausgestaltungen weisen die Querschnittsbilder aber anstelle der kreisrunden Außenkontur andere Formationen entsprechend den Fig. 7 bis 9 auf; die dreidimensionale Form derartiger Körper nach Fig. 1 kann daher sehr unterschiedlich sein, je nach der ausgewählten Dimensionierung und Ausgestaltung der dritten Achse.
Mit 12 ist eine Querschnittsebene des Körperchens bezeichnet, deren verschiedenartige Ausgestaltungen in den Fig. 7 bis 9 dargestellt sind.

In Fig. 2 ist ein axiales Längsschnittbild eines Körperchens dargestellt; die punktierten Linien definieren wieder die drei Abschnitte, wobei hier unterschiedliche Verjüngungsabschnitte vorliegen, die durch den Draht/Faden 7 in je zwei Segmente unterteilt werden. Der linke Verjüngungsabschnitt wird durch die beiden, im dargestellten Fall spiegelbildlichen Segmente 8a und 8b gebildet und besitzt eine kreisrunde Außenkontur. Der rechtsständige Verjüngungsabschnitt setzt sich aus den ebenfalls spiegelbildlichen Segmenten 10a und 10b zusammen, sodaß die Außenkonturen dieses zweiten Verjüngungsabschnitts einem Ellipsensegment entsprechen.

Es sei hier ausdrücklich darauf hingewiesen, daß sich die erfindungsgemäßen Ausgestaltungen nicht darauf beschränken, daß die Verjüngungsabschnitte aus spiegelbildlichen oder gleichartigen Segmenten bestehen, vielmehr können sich die Verjüngungsabschnitte aus unterschiedlichen Segmenten zusammensetzen.
Zwischen den beiden Verjüngungsabschnitten befindet sich der bauchige Mittelabschnitt, dessen beide Außenkonturen 9a und 9b in diesem Fall spiegelbildlich sind und Ellipsensegmente darstellen.

Mit 12 ist eine Querschnittsebene des Körperchens bezeichnet, deren verschiedenartige Ausgestaltungen in den Fig. 7 bis 9 dargestellt sind.

In Fig. 3 unterteilen die punktierten Linien im axialen Längsschnittsbild das pharmakafreisetzende Körperchen wieder in drei Abschnitte, und mit 12 wird eine Querschnittsebene des Körperchens bezeichnet, deren verschiedenartige Ausgestaltungen in den Fig. 7 bis 9 dargestellt sind. Der linksseitige Verjüngungsabschnitt besteht aus dem konischen Segment 13a und dem konkaven Segment 13b, ist in Bezug auf die Faden-/Drahtachse nicht spiegelbildlich und bildet einen rechtwinklig zur Draht-Fadenachse abgeschnittenen Stumpf. Der rechtsseitige Verjüngungsabschnitt wird durch die beiden konischen Segmente 15a und 15b gebildet und läuft spitz aus. Zwischen den beiden Verjüngungssegmenten befindet sich der mittig querschnittsreduzierte Mittelabschnitt, der durch die unterschiedlichen Außenkonturen 14a und 14b definiert ist.
Bei dieser Ausbildung setzen sich also sowohl ein Verjüngungsabschnitt wie auch der Mittelabschnitt aus je zwei unterschiedlichen und nicht spiegelbildlichen Segmenten zusammen.

Das in Fig. 4 im axialen Längsschnittbild dargestellte Körperchen entspricht in dieser Projektion einer Ellipse, wobei die Außenkonturen der Partikelhälften 16a und 16b spiegelbildlich sind. Die Außenkonturen 16a und 16b der Körperchen können aber auch asymmetrisch sein und Segmente unterschiedlicher Funktionen darstellen.
Mit 12 ist eine Querschnittsebene des Körperchens bezeichnet, deren verschiedenartige Ausgestaltungen in den Fig. 7 bis 9 dargestellt sind.
In das Körperchen sind leicht aus dem Zentrum versetzt die drei Hauptachsen a, b und c eingezeichnet, die die räumliche Ausbildung beschreiben.

Fig. 5 stellt ein pharmakafreisetzendes Körperchen dar, das im Längsschnittbild kreisrund gestaltet ist, d.h. die die Länge und Höhe bezeichnenden Achsen a und b sind gleich groß. Ein Partikel mit diesem Längsschnittbild fällt nur dann unter die erfindungsgemäßen Körperchen, wenn die dritte Achse c ungleich zu a und b ist.
Mit 12 ist eine Querschnittsebene des Körperchens bezeichnet, deren verschiedenartige Ausgestaltungen in den Fig. 7 bis 9 dargestellt sind.

In Fig. 6 ist ein kreisrunder Querschnitt eines pharmakafreisetzenden Körperchens gemäß den Querschnittsebenen 12 in den Fig. 1 bis 3 dargestellt. Der zentral verlaufende Draht/Faden ist mit 7 bezeichnet.

In Fig. 7 ist ein Querschnitt eines abgeplatteten pharmakafreisetzenden Körperchens gemäß den Querschnittsebenen 12 in den Fig. 1 bis 5 dargestellt. Im Querschnittsbild sind die beiden Enden durch Kreisbögen charakterisiert, an die sich ein rechteckiges Zwischenstück anschließt. Die lange Achse kann dabei entweder a oder b gemäß dem dreidimensionalen Achsensystem in Fig. 4 sein.
Der zentral verlaufende Draht/Faden ist mit 7 bezeichnet.

In Fig. 8 ist ein elliptischer Querschnitt eines pharmakafreisetzenden Körperchens gemäß den Querschnittsebenen 12 in den Fig. 1 bis 5 dargestellt. Die lange Achse kann dabei entweder a oder b gemäß dem dreidimensionalen Achsensystem in Fig. 4 sein. Der zentral verlaufende Draht/Faden ist mit 7 bezeichnet.

In Fig. 9 ist ein fast rechteckiger Querschnitt eines pharmakafreisetzenden Körperchens gemäß den Querschnittsebenen 12 in den Fig. 1 bis 5 dargestellt, bei dem nur die Ecken abgerundet sind. Die lange Achse kann dabei entweder a oder b gemäß dem dreidimensionalen Achsensystem in Fig. 4 sein. Der zentral verlaufende Draht/Faden ist mit 7 bezeichnet.

Die erfindungsgemäßen Formausgestaltungen der pharmakafrei setzenden Körperchen ergeben sich aus den Kombinationsmöglichkeiten der unterschiedlichen Abschnitte im Längschnittbild mit verschiedenen Querschnittformen. Die Verjüngungsabschnitte können sich wiederum aus einer Vielzahl verschiedener Segmente zusammensetzen, die frei miteinander kombinierbar sind. Diese Kombinationsmöglichkeiten treffen auch für die Mittelabschnitte zu.

Es wird an dieser Stelle noch einmal ausdrücklich darauf hingewiesen, daß sich die erfindungsgemäßen Ausgestaltungen nicht auf die in den Zeichnungen dargestellten Formen beschränken.

## Patentansprüche

1. Pharmakafreisetzende Körperchen zur lokalen Verwendung im oder auf dem Körpergewebe, bestehend aus einen biologisch inerten Trägermaterial, in dem ein oder verschiedene Pharmaka in gleichförmiger Verteilung und freisetzbarer Form vorliegen, wobei die Körperchen durch Fäden oder Drähte miteinander verbunden sind, dadurch gekennzeichnet, daß die Körperchen eine von der reinen Rotationsellipsoidform abweichende, an beiden Draht- oder Fadenaustrittstellen sich verjüngende Formgestalt und in beiden endständigen Verjüngungsabschnitten zumindest in einer Querschnittsebene eine gekrümmte Außenkontur aufweisen.

2. Pharmakafreisetzende Körperchen nach Anspruch 1, dadurch gekennzeichnet, daß die Körperchen in mindestens einem axialen Längsschnittbild aus mindestens zwei verschiedenen Abschnitten (4; 5) zusammengesetzt sind, deren Außenkonturen durch Kurven unterschiedlicher Funktionen definiert sind.

3. Pharmakafreisetzende Körperchen nach Anspruch 2, dadurch gekennzeichnet, daß die Körperchen in mindestens einem axialen Längsschnittbild aus mindestens drei verschiedenen Abschnitten (4; 5; 6) zusammengesetzt sind, deren Außenkonturen durch Kurven mit mindestens zwei unterschiedlichen Funktionen definiert sind.

4. Pharmakafreisetzende Körperchen nach einem oder mehreren der vorangehenden Ansprüche, dadurch gekennzeichnet, daß die Körperchen in mindestens einem axialen Querschnittbild aus mindestens zwei verschiedenen Abschnitten zusammengesetzt sind, deren Außenkonturen durch Kurven unterschiedlicher Funktionen definiert sind.

5. Pharmakafreisetzende Körperchen nach Anspruch 4, dadurch gekennzeichnet, daß die Körperchen in mindestens einem axialen Querschnittbild aus mindestens drei verschiedenen Abschnitten zusammengesetzt ist, deren Außenkonturen durch Kurven mit mindestens zwei unterschiedlichen Funktionen definiert sind.

6. Pharmakafreisetzende Körperchen nach einem oder mehreren der vorangehenden Ansprüche dadurch gekennzeichnet, daß die Körperchen zwischen den beiden endständigen Verjüngungsabschnitten (4; 6) einen im Längsschnittbild im wesentlichen walzenförmigen Mittelabschnitt (5) aufweisen.

7. Pharmakafreisetzende Körperchen nach einem oder mehreren der vorangehenden Ansprüche dadurch gekennzeichnet, daß die Körperchen zwischen den beiden endständigen Verjüngungsabschnitten (8; 10) einen im Längsschnittbild im wesentlichen faßförmig-bauchigen Mittelabschnitt (9) aufweisen.

8. Pharmakafreisetzende Körperchen nach einem oder mehreren der vorangehenden Ansprüche dadurch gekennzeichnet, daß die Körperchen zwischen den endständigen Verjüngungsabschnitten einen im Längsschnittbild konkaven Mittelabschnitt (14a) aufweisen.

9. Pharmakafreisetzende Körperchen nach einem oder mehreren der vorangehenden Ansprüche dadurch gekennzeichnet, daß die Körperchen zwischen den endständigen Verjüngungsabschnitten einen im Längsschnittbild im wesentlichen elliptischen Mittelabschnitt aufweisen.

10. Pharmakafreisetzende Körperchen nach einem oder mehreren der vorangehenden Ansprüche dadurch gekennzeichnet, daß die Körperchen im Längsschnittbild im wesentlichen durch eine Kurve zweiter Ordnung (14; 15) charakterisiert sind.

11. Pharmakafreisetzende Körperchen nach einem oder mehreren der vorangehenden Ansprüche dadurch gekennzeichnet, daß mindestens ein Segment mindestens eines endständigen Verjüngungsabschnitts (8; 10) im wesentlichen durch eine Kurve zweiter oder höherer Ordnung beschreibbar ist.

12. Pharmakafreisetzende Körperchen nach einem oder mehreren der vorangehenden Ansprüche dadurch gekennzeichnet, daß mindestens ein Segment mindestens eines endständigen Verjüngungsabschnitts (13a; 15a; 15b) in mindestens einem Längsschnitt konisch oder konoidisch ist.

13. Pharmakafreisetzende Körperchen nach einem oder mehreren der vorangehenden Ansprüche dadurch gekennzeichnet, daß mindestens einer der endständigen Verjüngungsabschnitte (13) konkav ist.

14. Pharmakafreisetzende Körperchen nach einem oder mehreren der vorangehenden Ansprüche dadurch gekennzeichnet, daß die Körperchen in zwei zu einander rechtwinklig versetzten axialen Längsschnittebenen unterschiedliche Außenkonturen aufweisen.

15. Pharmakafreisetzende Körperchen nach einem oder mehreren der vorangehenden Ansprüche dadurch gekennzeichnet, daß die Körperchen in Längsachse (1; Fig. 2; Fig. 3; Fig. 4) abgeplattet sind.

16. Pharmakafreisetzende Körperchen nach einem oder mehreren der vorangehenden Ansprüche dadurch gekennzeichnet, daß die Körperchen im Querschnitt (Fig. 7; Fig. 8; Fig. 9) eine von der Kreisform abweichende Form aufweisen.

17. Pharmakafreisetzende Körperchen nach Anspruch 12 dadurch gekennzeichnet, daß die Körperchen dreiachsige (a; b; c) Ellipsoide mit mindestens zwei unterschiedlichen Hauptachsen darstellen.

18. Pharmakafreisetzende Körperchen nach einem oder mehreren der vorangehenden Ansprüche dadurch gekennzeichnet, daß die Körperchen Ellipsoide mit drei unterschiedlichen Hauptachsen (a; b; c) darstellen.

19. Pharmakafreisetzende Körperchen nach einem oder mehreren der vorangehenden Ansprüche dadurch gekennzeichnet, daß die Körperchen eine gerillte oder gefurchte Oberfläche aufweisen.

20. Pharmakafreisetzende Körperchen nach einem oder mehreren der vorangehenden Ansprüche, dadurch gekennzeichnet, daß die Körperchen als Pharmaka anti-entzündliche und/oder anti-tumoröse Wirkstoffe, wie z.B. Antibiotika, Antseptika, Chemotherapeutika, Kortikoide, Antiphlogistika, Zytostatika, enthalten.

## Claims

1. Drug delivering corpuscles for locally application in or on the body tissue consisting of a biologically inert carrier material with one or various medicaments being present in homogenous distribution and capable of release, and the corpuscles being connected together by threads or wires, characterized in that the corpuscles have a shape which differs from the exact spheroid configuration, tapering end sections at both outlets of threads or wires and an arcuate outline in both tapering end section in at least one transverse plane.

2. Drug delivering corpuscles according to Claim 1, characterized in that the corpuscles in at least one axially longitudinal cutting plane consist of at least two different sections (4; 5), the outlines of them being determined by curvatures which are different from one another.

3. Drug delivering corpuscles according to Claim 2, characterized in that the corpuscles in at least one axially longitudinal cutting plane consist of at least three different sections (4; 5; 6), the outlines of them being determined by curvatures of at least two different functions.

4. Drug delivering corpuscles according to one or more of the precedent Claims, characterized in that the corpuscles in at least one axially transverse cutting plane consist of at least two different sections, the outlines of them being determined by curvatures which are different from one another.

5. Drug delivering corpuscles according to Claim 4, characterized in that the corpuscles in at least one axially transverse cutting plane consist of at least three different sections, the outlines of them being determined by curvatures of at least two different functions.

6. Drug delivering corpuscles according to one or more of the precedent Claims, characterized in that the corpuscles contain in a longitudinal cutting plane a roller-shaped middle section (5) between the both tapering end sections (4; 6).

7. Drug delivering corpuscles according to one or more of the precedent Claims, characterized in that the corpuscles contain a barrel-shaped middle section (9) between the both tapering end sections (8; 10).

8. Drug delivering corpuscles according to one or more of the precedent Claims, characterized in that the corpuscles contain a concave-shaped middle section (9) between the both tapering end sections (8; 10).

9. Drug delivering corpuscles according to one or more of the precedent Claims, characterized in that the corpuscles contain a substantially elliptical-shaped middle section between the both tapering end sections.

10. Drug delivering corpuscles according to one or more of the precedent Claims, characterized in that the outlines of the corpuscles in the axially longitudinal cutting plane constitute a curvature of second order (14; 15).

11. Drug delivering corpuscles according to one or more of the precedent Claims, characterized in that the outline of at least one segment of at least one tapering end section (8; 10) substantially constitute a curvature of second or higher order.

12. Drug delivering corpuscles according to one or more of the precedent Claims, characterized in that at least one segment of at least one tapering end section (13a; 15a; 15b) having a conical or conoidal cross-sectional outline in at least one longitudinal plane.

13. Drug delivering corpuscles according to one or more of the precedent Claims, characterized in that at least one of the tapering end sections (13) have a concave outline.

14. Drug delivering corpuscles according to one or more of the precedent Claims, characterized in that the corpuscles contain different outlines in two axial longitudinal cutting planes which are inclined at right angle relative to each other.

15. Drug delivering corpuscles according to one or more of the precedent Claims, characterized in that the corpuscles are flattened in the longitudinal cross-section (Fig. 1, Fig. 2; Fig. 3; Fig. 4).

16. Drug delivering corpuscles according to one or more of the precedent Claims, characterized in that the corpuscles have a cross-sectional outline departing from a circular cross-sectional outline (Fig. 7; Fig. 8; Fig. 9).

17. Drug delivering corpuscles according to claims 12, characterized in that the corpuscles constituting ellipsoids have three main axes and having different dimensions as measured in the direction of at least two of said axes.

18. Drug delivering corpuscles according to one or more of the precedent Claims, characterized in that the corpuscles constitute ellipsoids having three main axes and having different dimensions as measured in the direction of each of said axes.

19. Drug delivering corpuscles according to one or more of the precedent Claims, characterized in that the corpuscles have an external surface which is ribbed or grooved.

20. Drug delivering corpuscles according to one or more of the precedent Claims , characterized in that the corpuscles contain pharmaceuticals selected from anti-inflammatory and/or anti-tumourous agents, as antibiotics, antiseptics, chemotherapeutics, corticoids, antiphlogistics, cytostatics.

## Revendications

1. Corpuscules délivrant des médicaments pour l'application locale dans ou sur le tissu corporel, constitués d'une matière porteuse biologique inerte, où un ou différents médicaments libérables se trouvent régulièrement répartis, et reliés les uns aux autres par des fils ou fils métalliques, caractérisés en cc que les corpuscules présentent une forme s'écartant de la configuration d'un sphéroïde exacte, en ce que la forme des corpuscules se rétrécit près des deux sorties des fils et en ce que le corpuscules présentent dans les deux extrémités rétrécissants au moins dans un coupe transversal un contour incurvé.

2. Corpuscules délivrant des médicaments selon la revendication 1, caractérisés en ce que les corpuscules se composent au moins dans un coupe longitudinal axial au moins de deux segments (4 ; 5), leur contours sont définis par des courbes différentes.

3. Corpuscules délivrant des médicaments selon la revendication 2, caractérisés en ce que les corpuscules se composent au moins dans un coupe longitudinal axial au moins de trois segments différents (4 ; 5 ; 6), leur contours sont définis par au moins de deux courbes différents.

4. Corpuscules délivrant des médicaments selon une ou plusieurs des revendications précédentes, caractérisés en ce que les corpuscules se composent dans un coupe transversal axial au moins de deux segments, leur contours sont définis par des courbes différents.

5. Corpuscules délivrant des médicaments selon une ou plusieurs des revendication précédentes, caractérisés en ce que les corpuscules se composent au moins dans un coupe transversal axial au moins de trois segments différents, leur contours sont définis par au moins de deux courbes différents.

6. Corpuscules délivrant des médicaments selon une ou plusieurs des revendications précédentes, caractérisés en ce que les corpuscules présentent au milieu de deux extrémités rétrécissantes (4; 6) un segment médian ayant une forme essentiellement cylindrique (5).

7. Corpuscules délivrant des médicaments selon une ou plusieurs des revendications précédentes, caractérisés en ce que les corpuscules présentent au milieu de deux extrémités rétrécissantes (8; 10) un segment médian ayant une forme essentiellement renflée (9.

8. Corpuscules délivrant des médicaments selon une ou plusieurs des revendications précédentes, caractérisés en ce que les corpuscules présentent au milieu de deux extrémités rétrécissantes un segment médian ayant une forme concave (14a).

9. Corpuscules délivrant des médicaments selon une ou plusieurs des revendications précédentes, caractérisés en ce que les corpuscules présentent au milieu de deux extrémités rétrécissantes un segment médian ayant une forme essentiellement elliptique.

10. Corpuscules délivrant des médicaments selon une ou plusieurs des revendications précédentes, caractérisés en ce que les corpuscules sont déterminés dans un coupe transversale essentiellement par une courbe de deuxième ordre (14 ; 15).

11. Corpuscules délivrant des médicaments selon une ou plusieurs des revendications précédentes, caractérisés en ce que au moins un segment au moins d'une extrémité rétrécissante (8 ; 10) est déterminé essentiellement par une courbe d'ordre seconde ou surélevé (14 ; 15).

12. Corpuscules délivrant des médicaments selon une ou plusieurs des revendications précédentes, caractérisés en ce que au moins un segment au moins d'une extrémité rétrécissante (13a ; 15a 15b) est conique ou conoïde au moins dans un coupe longitudinale.

13. Corpuscules délivrant des médicaments selon une ou plusieurs des revendications précédentes, caractérisés en ce que au moins une extrémité rétrécissante est concave (13).

14. Corpuscules délivrant des médicaments selon une ou plusieurs des revendications précédentes, caractérisés en ce que les corpuscules présentent en deux coupes longitudinaux axiales, qui sont déplacés à l'angle droit l'un vers l'autre, des contours différents.

15. Corpuscules délivrant des médicaments selon une ou plusieurs des revendications précédentes, caractérisés en ce que les corpuscules sont aplatis dans l'axe longitudinal (1 ; Fig. 2 ; Fig. 3 ; Fig.4).

16. Corpuscules délivrant des médicaments selon une ou plusieurs des revendications précédentes, caractérisés en ce que les corpuscules présentent dans un coupe transversal (Fig. 7 ; Fig. 8 ; Fig. 9) une forme s'écartant de la forme sphérique.

17. Corpuscules délivrant des médicaments selon la revendication 12, caractérisés en ce que les corpuscules établissent des éllipsoïdes trois-axiques (a ; b ; c) avec au moins deux axes principaux différents.

18. Corpuscules délivrant des médicaments selon une ou plusieurs des revendications précédentes, caractérisés en ce que les corpuscules établissent des ellipsoïdes avec trois axes principaux différents (a ; b ; c).

19. Corpuscules délivrant des médicaments selon une ou plusieurs des revendications précédentes, caractérisés en ce que les corpuscules présentent une surface sillonnées ou ridées.

20. Corpuscules délivrant des médicaments selon une ou plusieurs des revendications précédentes, caractérisés en ce que les corpuscules renferment des pharmaceutiques, qui sont des substances anti-infecteuses et/ou anti-tumeureuses, par example des substances antibiotiques, antiseptiques, chimiothérapeutiques, antiphlogistiques, cytostatiques ou des corticoides.
